# EUROPEAN PATENT SPECIFICATION

(11) **EP 3 611 250 B1**
(45) Date of publication and mention of the grant of the patent: **22.05.2024**
(21) Application number: 18792326.3
(22) Date of filing: 18.04.2018
(51) Int. Cl.: C12M 1/107, C12M 1/02, C12M 1/00, C12P 5/02, C12M 1/26

(54) **FERMENTATION EQUIPMENT USED FOR ORGANIC MATTER AND METHOD THEREFOR**
FERMENTATIONSANLAGE VERWENDET FÜR ORGANISCHE STOFFE UND VERFAHREN DAFÜR
ÉQUIPEMENT DE FERMENTATION UTILISÉ POUR LA MATIÈRE ORGANIQUE ET MÉTHODE ASSOCIÉE

(30) Priority: 26.04.2017 CN 201710280353
(43) Date of publication of application: 19.02.2020
(73) Proprietor: Shanghai Beiqi New Energy Technology Co., Ltd., Shanghai 200444 (CN)
(72) Inventor: STEPHANE, Vernede, Changzhou Jiangsu 213017 (CN)
(74) Representative: Sarpi, Maurizio
(86) International application number: PCT/CN2018/083514
(87) International publication number: WO 2018/196666

(56) References cited:
- WO-A1-95/32158
- WO-A1-2015/058295
- CN-A- 1 121 343
- CN-A- 102 965 278
- CN-A- 103 045 651
- CN-A- 107 034 126
- CN-U- 205 635 580
- CN-U- 205 710 725
- DE-A1-102013 114 786
- DE-U1- 20 121 701
- US-A1- 2011 281 254

## Description

### Technical Field

The present invention relates to a device and method for treatment of organic matter, in particular a device and method for the digestion of organic matter.

### BACKGROUND OF THE INVENTION

Digestion of organic matter is an important technology for handling solid and liquid organic waste, and is also one of the sources of renewable energy and organic fertilizers. Digestion of organic matter technologies are basically classified into three types: anaerobic digestion of sewage where the dry matter content is less than 5%; wet digestion where the dry matter content is within 5%-20%; and dry digestion where the dry matter content exceeds 20%.

Wet digestion is usually using a completely mixed wet digestion process carried out in a completely mixed anaerobic reactor. In such a reaction device, the retention time of the organic matter is at least 30 days. If the retention time of the organic matter is less than 30 days, when the organic matter are discharged they will produce a wash out of the microorganisms. Moreover, the complete digestion of organic matters requires more time, about 90 days. Therefore, this technology needs a very large reaction device, which represents a high investment cost and operating cost. Besides, this technology is very sensitive to organic loads, and variations in the organic loads may result in a digestion imbalance and even a stoppage.

Dry digestion is classified into continuous dry digestion and batch dry digestion. For continuous dry digestion technology, organic matter is inserted at one end of the reaction device, and flow toward the other end in a piston flow fashion. The reaction device may be horizontal or vertical. The retention time of organic materials is usually 20 days. After 20 days, the digestion is not fully completed, and the liquid phase is usually transported to a completely mixed anaerobic reaction device to finalize digestion. The capital investment of the continuous dry digestion process is higher than that of the wet digestion process, as some specialized equipment are required. The operation of the continuous dry digestion process also requires a significant know-how. In the batch dry digestion process, organic matter enters the reaction device by batch and then has a liquid poured over them, the percolated liquid is then collected to perform pouring cycles. The retention time of each batch is about 60 days. The project investment cost of the batch dry digestion process is a little lower than that of the continuous dry digestion process. However, the digestion is implemented by batch, so the operation of the batch dry digestion process is very complicated.

Some anaerobic digestion processes adopt two steps. The first step is acidification in a first reactor. The organic matter in this first reactor has a relatively high solid content. The second step is digestion implemented in a second reactor. The organic matter in this second reactor has relatively low solid content. In such process, a part of the liquid in the second reactor is circulated into the first reactor. However, such process has a major defect that the liquid cannot be effectively circulated in the acidification reactor because of the very low permeability of materials in the acidification reactor. The efficiency of such process is greatly affected.

In order to realize wider application, the anaerobic digestion technology must overcome the following difficulties: 1. reducing investment cost and operating cost to improve economic appeal; 2. reducing the reactor volume to use the reactor in places with limited space; 3. enhancing digestion efficiency to increase energy source yield and reduce pollution; 4. improving process stability to ensure production stability.

DE102013114786A1 relates to a method for producing biogas and a biogas plant (1) for wet fermentation by use of nitrogen-rich, interfering-substance-laden input substrate (3) having an ammonium nitrogen concentration of more than 5 kg NH4-N/m3, wherein a first process stage (2) is provided for interfering-substance separation and a second process stage (12) is provided for the continuous formation of acetic acid and methane at a substantially stable temperature in the psychrophilic range. The invention further relates to a fermentation tank (5, 10, 18) of a biogas plant (1), wherein the fermentation tank (5, 10, 18) has a vertically arranged screw (25) provided with a rotary drive, which screw runs in a pipe (26) that is open at both ends and substantially fills up the pipe (26), wherein the pipe (26) extends from below the substrate level to above the bottom (22) of the fermentation tank (5, 10, 18) and is firmly connected to the fermentation tank (5, 10, 18).

### BRIEF SUMMARY OF THE INVENTION

In view of the above-mentioned defects in the prior arts, the technical problem to be solved by the present invention is to provide a fermentation equipment used for organic matter and method therefor, realizing efficient digestion in a relatively short retention time.

To fulfill the above-mentioned objective, the present invention provides a fermentation equipment used for organic matter according to claims 1-4.

The present invention also provides a fermentation method used for organic matter according to claims 5-11.

As a result, the fermentation equipment used for organic matter and method therefor of the present invention realize full hydrolysis and acidification of organic materials in a short time. Moreover microorganisms are effectively retained in the second reaction device and allow to operate digestion with short residence time without wash out of the microorganism population. Compared with the traditional anaerobic digestion technology, the organic matter is fully decomposed, and a higher biogas yield is obtained. At the same time, a solid-liquid separation device is not needed, and a solid-liquid separation can be implemented in the first reaction device through the differential pressure device, the high-solid-content discharging device, and low-solid-content discharging device.

The concept, specific structure and generated technical effects of the present invention are further described below in conjunction with the attached drawings to fully explain the purpose, characteristics, and effects of the present invention.

### BRIEF DESCRIPTION OF THE SEVERAL VIEWS OF THE DRAWINGS

FIG. 1 is a schematic view of a device for organic digestion, which is present for illustration purposes only;
FIG. 2 is a schematic view of a second embodiment of the device for organic digestion, which is present for illustration purposes only;
FIG. 3 is a schematic view of a third embodiment of the device for organic digestion, according to the present invention.

### DETAILED DESCRIPTION OF THE INVENTION

The present invention is described in further detail in conjunction with the attached drawings.

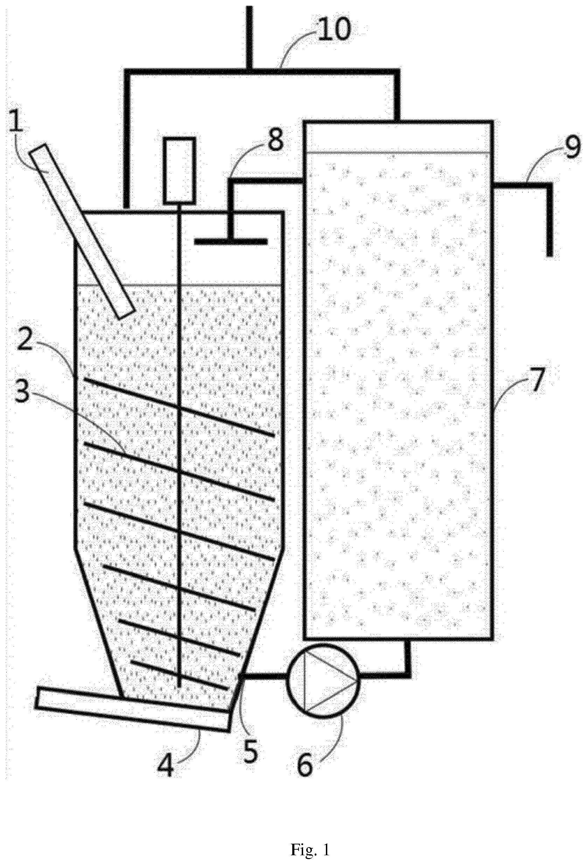

As shown in FIG.1, it is disclosed an embodiment which is not part of the present invention, wherein it is provided a fermentation equipment used for organic matter, including a feeding device 1, a first reaction device 2, an differential pressure device 3, a high-solid-content discharging device 4, a low-solid-content discharging device 5, a first cycle pump 6, a second reaction device 7, a first recirculation device 8, a first discharging device 9 and a collecting device 10, wherein the feeding device 1 is connected with the first reaction device 2 for conveying organic materials into the first reaction device 2; materials are fermented in the first reaction device 2; the differential pressure device 3 is installed in the first reaction device 2 for generating a pressure, release or traction on the materials; the high-solid-content discharging device 4 is connected with the first reaction 2 for discharging a material flow with a high solid content from the first reaction device 2; the low-solid-content discharging device 5 is connected with the first reaction 2 for discharging a material flow with a low solid content from the first reaction device 2; the first cycle pump 6 is arranged between the low-solid-content discharging device 5 and the second reaction device 7 for conveying the material flow with a low solid content into the second reaction device 7; materials are fermented in the second reaction device 7, and the solid content of the second reaction device 7 is smaller than that of the first reaction device 2; the recirculation device 8 is arranged between the first reaction device 2 and the second reaction device 7 for recirculating the materials in the second reaction device 7 back into the first reaction device 2; the first discharging device 9 is connected with the second reaction device 7; the collecting device 10 is respectively connected with the first reaction device 2 and the second reaction device 7 for collecting the biogas generated by the first reaction device 2 and the second reaction device 7. It needs to be noted that the material flow with high solid content and the material flow with low solid content are relative term, the actual solid content of those two flows can be adjusted according to the type of organic matter to be digested.

When the differential pressure device applies a pressure onto the materials, liquid easily flows out of the solids. When the applied pressure is reduced, the liquid is re-mixed with the solids. When the differential pressure device applies a traction to the materials, liquid is absorbed out of the solids. Pressurization, decompression, and traction drives the liquid to efficiently circulate in solids, so that the materials in the first reaction device 2 have an high permeability. The differential pressure device 3 needs one hour or less time to complete an operating cycle which includes the procedures of generating a pressure first, then reducing the pressure and finally generating a traction. Specifically, the time can be set according to the actual situations, within a range of 1 min-1h, for example, 15 min, 5 min or 1 min. In this example the differential pressure device 3 has an Archimedes screw structure. It needs to be noted that this differential pressure device 3 can also be realized by a compressor structure or a hydraulic piston device.

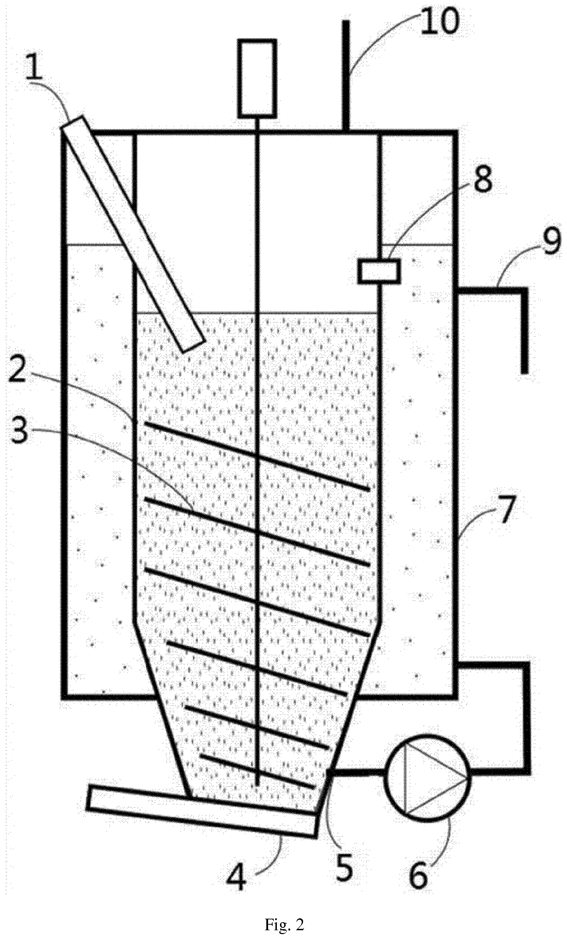

The first reaction 2 can be vertical, with the plug flow anaerobic digestion process. Materials are fed via the upper part and discharged via the lower part. The second reaction device 7 can use a high-load organic waste water process, for example, an upflow anaerobic sludge blanket reactor, and an efficient anaerobic sludge blanket reactor. It need to be noted that, as shown in FIG.2 relating an embodiment which is not part of the present invention, the second reaction device 7 and the first reaction device 2 may be separated with a partition board in the same reaction tank.

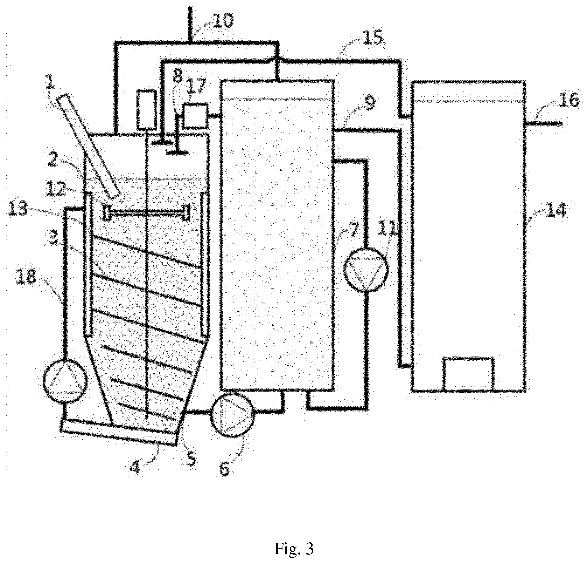

As shown in FIG.3, according to the present invention the fermentation equipment used for organic matter can also include a second cycle pump 11, and the second cycle pump 11 circulates the materials in the second reaction device 7.

The fermentation equipment used for organic matter can also include a stirring device 12, and the stirring device 12 is arranged in the first reaction device 2 for stirring materials.

The fermentation equipment used for organic matter can also include a heating device 13, and the heating device 13 may be arranged at any position and can use hot water or steam circulation.

The fermentation equipment used for organic matter can also include a third reaction device 14, a second recirculation device 15, and a second discharging device 16, wherein the third reaction device 14 is connected with the second reaction device 7 through the first discharging device 9; the materials in the second reaction device 7 are conveyed into the third reaction device 14 by the first discharging device 9; the second recirculation device 15 is arranged between the third reaction device 14 and the first reaction device 2 for recirculating the materials in the third reaction device 14 back into the first reaction device 2; and the second discharging device 16 is connected with the third reaction device 14. The third reaction device 14 can drive the materials to react more effectively and enhance the reaction efficiency. It needs to be noted that when the materials in the second reaction device 7 are all conveyed into the third reaction device 14 by the first discharging device 9, the first recirculation device 8 may not be used for recirculating materials any longer, and may be removed.

The fermentation equipment used for organic matter also includes a dosing device 17; the dosing device 17 is arranged at any position in the first reaction device 2, the second reaction device 7, the third reaction device 14, the first recirculation device 8 or the second recirculation device 15 for controlling the pH value in the first reaction device 2, the second reaction device 7 and the third reaction device 14, and realizing crystallization of nitrogen and/or phosphorus.

The fermentation equipment used for organic matter can also include a third recirculation device 18. This third recirculation device 18 recirculate materials with a high solid content into the first reaction device 2 to drive the materials to react fully.

It needs to be noted that the number of each devices in the present fermentation equipment used for organic matter can be set according to actual situations, and their number is not limited to one.

A fermentation method used for organic matter includes the following steps: (1) conveying materials into the first reaction device 2 by the feeding device 1 to perform a fermenting reaction; (2) inducing a relative flow of the solid phase and liquid phase in the first reaction device with the differential pressure device by creating cycles of excess pression, decompression and/or traction on the solid phase relatively to the liquid phase; (3) discharging the high-solid-content material flow from the first reaction device 2 by the high-solid-content discharging device 4, discharging the low-solid-content material flow from the first reaction device 2 by the low-solid-content discharging device 5 and pumping the low-solid-content material flow into the second reaction device 7 by the first cycle pump 6 to perform a digestion reaction; (4) recirculating the materials in the second reaction device 7 back into the first reaction device 2 by the first recirculation device 8, mixing the materials with the original material in the first reaction device 2, and repeating step (2).

The duration of one cycle of the differential pressure device 3 is 1 h, 15 min or a shorter time period.

In step (1), materials continuously enter the first reaction device 2; and in step (3), materials are continuously discharged from the first reaction device 2.

In step (1), the materials are conveyed by the feeding device 1 at an interval of 1-60 min.

In step (3), the high-solid-content material flow is discharged from the first reaction device 2 by the high-solid-content discharging device 4 at an interval of 1-60 min.

The first reaction device 2 is vertical; in step (1), materials are fed via the upper part, and in step (3), the materials are discharged via the lower part. As an alternative embodiment, the first reaction device is vertical; in step (1), the materials are fed via the lower part, and in step (3), the high-solid-content material flow is charged via the upper part, and the low-solid-content material flow is charged via the lower part.

The solid content of the first reaction device 2 is maintained to be greater than 12%, and the solid content of the second reaction device 7 is maintained to be lower than 12%.

The second reaction device 7 adopts an anaerobic digestion process.

The temperature of the first reaction device 2 and the second reaction device 7 is maintained between 30-55°C.

Step (4) also includes the following sub-steps: a. the materials in the second reaction device 7 are discharged and driven to flow to the third reaction device 14 by the first discharging device 9, to perform an aerobic digestion reaction; b. the materials in the third reaction device 14 are driven to flow back into the first reaction device 2 by the second recirculation device 15 to be introduced in the first reaction device 2, and then step (2) is repeated.

Step (3) also includes the following sub-step: the high-solid-content material flow is extracted from the first reaction device 2, and then is driven to re-enter the first reaction device 2 by the third recirculation device 18 to be mixed with the original materials in the first reaction device.

The solid content in the first reaction device 2 can be controlled according to the running cycle of the differential pressure device 3 and the flow rate of the low-solid-content material flow pumped by the cycle pump 6 to obtain an appropriate reaction environment.

According to this method, soluble substances are efficiently extracted from the first reaction device 2, thus inducing a full hydrolysis and acidification of organic materials in a short time. Compared with the traditional anaerobic digestion technology, the organic matter is fully decomposed, and a higher biogas yield is obtained. This yield can be up to 20% higher than the yield of the traditional digestion process. At the same time, a solid-liquid separation device is not needed. The solid-liquid separation is automatically implemented in the first reaction device 2 by the differential pressure device 3, the high-solid-content discharging device 4, and low-solid-content discharging device 5.

The high-solid-content materials and the low-solid-content materials are respectively discharged by the high-solid-content discharging device 4 and the low-solid-content discharging device 5.

The materials in the first reaction device 2 flow into the second reaction device 7, and the materials in the second reaction device 7 flow into the first reaction device 2. Those flow can be implemented simultaneously to ensure that the device is always in an optimal digestion state. The second reaction device 7 can efficiently retain microorganisms for digestion, this allows this method to realize a complete digestion in a relatively retention short time. The existing dry digestion or completely mixed wet type digestion fails to realize digestion in such short time as microorganisms are discharged out together with the fermented materials. According to the method, the mean retention time of the materials is within 10 days. The volume of the required reaction device is much smaller than that the digestion tanks for the traditional digestion process, thereby greatly reducing the investment cost and lowering the land requirements for the process. Another advantage of the method is stability of system operation. The response to the increase in the organic loads is stable. Therefore, the operational management of the method is easier.

## Claims

1. A fermentation equipment used for organic matter, **characterized by** comprising a feeding device (1), a first reaction device (2), a differential pressure device (3), a high-solid-content discharging device (4), a low-solid-content discharging device (5), a first cycle pump (6), a second reaction device (7), a first recirculation device (8), a first discharging device (9), a collecting device (10), and a stirring device (12), a third recirculation device (18), wherein the feeding device (1) is connected with the first reaction device (2); the stirring device (12) is arranged in the first reaction device (2); the differential pressure device (3) is installed in the first reaction device (2); the high-solid-content discharging and the low-solid content discharging device are respectively connected with the first reaction device (2); the first cycle pump (6) is arranged between the low-solid content discharging device and the second reaction device (7); the first recirculation device (8) is arranged between the first reaction device (2) and the second reaction device (7); the first discharging device (9) is connected with the second reaction device (7); and the collecting device (10) is respectively connected with the first reaction device (2) and the second reaction device (7) for collecting the biogas generated in said first and second reaction devices (2, 7); the third recirculation device (18) is connected to the first reaction device (2) and the high-solid-content discharging device (4), to recirculate materials with a high solid content into the first reaction device (2) to drive the materials to react fully, the differential pressure device (3) is an Archimedes screw structure, or a hydraulic piston device, or a compressor structure.

2. The fermentation equipment used for organic matter according to claim 1, **characterized by** also comprising a heating device.

3. The fermentation equipment used for organic matter according to claim 1, **characterized by** also comprising a third reaction device, a second recirculation device, and a second discharging device, wherein the third reaction device is connected with the second reaction device (7) through the first discharging device (9); the second recirculation device is arranged between the third reaction device and the first reaction device (2); and the second discharging device is connected with the third reaction device.

4. The fermentation equipment used for organic matter device according to claim 1, **characterized by** also comprising a dosing device, wherein the dosing device is arranged at any position in the first reaction device (2), the second reaction device (7), the third reaction device, the first recirculation device (8) or the second recirculation device.

5. A fermentation method used for organic matter, comprising the following steps:
(A) conveying materials into the first reaction device (2) by the feeding device (1) to perform a fermenting reaction;
(B) inducing a relative flow of the solid phase and liquid phase in the first reaction device (2) with the differential pressure device (3) by creating cycles of excess pression, decompression and/or traction on the solid phase relatively to the liquid phase the differential pressure device (3) is an Archimedes screw structure or a hydraulic piston device;
(C) discharging the high-solid-content material flow from the first reaction device (2) by the high-solid-content discharging device (4) and then the high-solid-content material flow is driven to re-enter the first reaction device (2) by the third recirculation device (18) to be mixed with the original materials in the first reaction device (2), discharging the low-solid-content material flow from the first reaction device (2) by the low-solid-content discharging device (5) and pumping the low-solid-content material flow into the second reaction device (7) by the first cycle pump (6) to perform a digestion reaction;
(D) recirculating the materials in the second reaction device (7) back into the first reaction device (2) by the first recirculation device (8), mixing the materials with the original material in the first reaction device (2), and repeating step (B); wherein it is further provided to recirculate materials with a high solid content into the first reaction device (2), by a third recirculation device (18), to drive the materials to react.

6. The fermentation method used for organic matter according to claim 5, **characterized in that** the running time of one cycle of the differential pressure device (3) is within the range of 1-60 min.

7. The fermentation method used for organic matter according to claim 5, **characterized in that** in step (A), materials continuously enter the first reaction device (2); and in step (C), materials are continuously discharged from the first reaction device (2).

8. The fermentation method used for organic matter according to claim 5, **characterized in that** in step (A), materials are conveyed by the feeding device (1) at an interval of 1-60 min; in step (C), the high-solid-content material flow is discharged out of the first reaction device (2) by the high-solid-content discharging device (4) at an interval of 1-60 min.

9. The fermentation method used for organic matter according to claim 5, **characterized in that** the solid content of the first reaction device (2) is maintained to be greater than 12%, and the solid content of the second reaction device (7) is maintained to be lower than 12%.

10. The fermentation method used for organic matter according to claim 5, **characterized in that** the temperature of the first reaction device (2) and the second reaction device (7) is maintained between 30-55 °C.

11. The fermentation method used for organic matter according to claim 5, **characterized in that** step (D) also comprises the following sub-steps:
(E) the materials in the second reaction device (7) are discharged and driven to flow to the third reaction device by the first discharging device (9);
(F) the materials in the third reaction device are driven to flow back into the first reaction device (2) by the second recirculation device to be mixed with the original materials in the first reaction device (2), and then step (B) is repeated.

## Patentansprüche

1. Fermentationsanlage für organische Stoffe, **dadurch gekennzeichnet, dass** sie eine Fördervorrichtung (1), eine ersten Reaktor (2), eine Differenzdruckvorrichtung (3), eine Auslassvorrichtung (4) für hohe Feststoffanteile, eine Auslassvorrichtung (5) für niedrige Feststoffanteile und eine erste Umlaufpumpe (6), einen zweiten Reaktor (7), einen ersten Rücklauf (8), eine erste Auslassvorrichtung (9), eine Auffangvorrichtung (10), und eine Rührvorrichtung (12), einen dritten Rücklauf (18) umfasst, wobei die Fördervorrichtung (1) mit dem ersten Reaktor (2) verbunden ist; wobei die Rührvorrichtung (12) in dem ersten Reaktor (2) angeordnet ist; wobei die Differenzdruckvorrichtung (3) in dem ersten Reaktor (2) installiert ist; wobei die Auslassvorrichtung für hohe Feststoffanteile und die Auslassvorrichtung für niedrige Feststoffanteile jeweils mit dem ersten Reaktor (2) verbunden sind; wobei die erste Umlaufpumpe (6) zwischen der Auslassvorrichtung für niedrige Feststoffanteile und dem zweiten Reaktor (7) angeordnet ist; wobei der erste Rücklauf (8) zwischen dem ersten Reaktor (2) und dem zweiten Reakt (7) angeordnet ist; wobei der erste Rücklauf (9) mit dem zweiten Reaktor (7) verbunden ist; und die Auffangvorrichtung (10) entsprechend mit dem ersten Reaktor (2) und dem zweiten Reaktor (7) verbunden ist, um das in dem ersten und zweiten Reaktor (2, 7) erzeugte Biogas zu sammeln; wobei der dritte Rücklauf (18) mit dem ersten Reaktor (2) und der Auslassvorrichtung (4) für hohe Feststoffanteile verbunden ist, um Materialien mit einem hohen Feststoffanteil in dem ersten Reaktor (2) zu rezirkulieren, um die Materialien zur vollständigen Reaktion zu bringen, wobei die Differenzdruckvorrichtung (3) eine archimedische Schraubenstruktur oder eine hydraulische Kolbenvorrichtung oder eine Verdichterstruktur ist.

2. Fermentationsanlage für organische Stoffe nach Anspruch 1, **dadurch gekennzeichnet, dass** sie ferner eine Heizvorrichtung umfasst.

3. Fermentationsanlage für organische Stoffe nach Anspruch 1, **dadurch gekennzeichnet, dass** sie ferner einen dritten Reaktor, einen zweiten Rücklauf und eine zweite Auslassvorrichtung umfasst, wobei der dritte Reaktor über die erste Auslassvorrichtung (9) mit dem zweiten Reaktor (7) verbunden ist; wobei der zweite Rücklauf zwischen dem dritten Reaktor und dem ersten Reaktor (2) angeordnet ist; und die zweite Auslassvorrichtung mit dem dritten Reaktor verbunden ist.

4. Fermentationsanlage für organische Stoffe nach Anspruch 1, **dadurch gekennzeichnet, dass** sie ferner eine Dosierungsvorrichtung umfasst, wobei die Dosierungsvorrichtung an einer beliebigen Position in dem ersten Reaktor (2), dem zweiten Reaktor (7), dem dritten Reaktor, dem ersten Rücklauf (8) oder dem zweiten Rücklauf angeordnet ist.

5. Fermentationsverfahren für organischen Stoff, das die folgenden Schritte umfasst:
(A) Zuführen von Materialien in den ersten Reaktor (2) durch die Fördervorrichtung (1), um eine Fermentationsreaktion durchzuführen;
(B) Einleiten einer relativen Strömung der festen Phase und der flüssigen Phase in dem ersten Reaktor (2) mit der Differenzdruckvorrichtung (3) durch Erzeugen von Überdruckzyklen, Dekompression und/oder Zug auf die feste Phase relativ zu der flüssigen Phase, wobei die Differenzdruckvorrichtung (3) eine archimedische Schraubenstruktur oder eine hydraulische Kolbenvorrichtung ist;
(C) Ablassen des Materialstroms mit hohem Feststoffanteil aus dem ersten Reaktor (2) durch die Auslassvorrichtung (4) für hohe Feststoffanteile, und anschließend wird der Materialstrom mit hohem Feststoffanteil angetrieben, um durch den dritten Rücklauf (18) wieder in den ersten Reaktor (2) einzudringen, um mit den ursprünglichen Materialien in dem ersten Rücklauf (2) gemischt zu werden, Ablassen des Materialstroms mit niedrigem Feststoffanteil aus dem ersten Reaktor (2) durch die Auslassvorrichtung (5) für niedrige Feststoffanteile und Pumpen des Materialstroms mit niedrigem Feststoffanteil in den zweiten Reaktor (7) durch die erste Umlaufpumpe (6), um eine Fermentationsreaktion durchzuführen;
(D) Rückführen der Materialien in den zweiten Reaktor (7) zurück in den erste Reaktor (2) durch den ersten Rücklauf (8), Mischen der Materialien mit dem ursprünglichen Material in dem ersten Reaktor (2) und Wiederholen von Schritt (B); wobei ferner vorgesehen ist, Materialien mit einem hohen Feststoffanteil in dem erste Reaktor (2) durch einen dritten Rücklauf (18) zurückzuführen, um die Materialien zum Reagieren zu bringen.

6. Fermentationsverfahren für organische Stoffe nach Anspruch 5, **dadurch gekennzeichnet, dass** die Laufzeit eines Zyklus der Differenzdruckvorrichtung (3) im Bereich von 1-60 min liegt.

7. Fermentationsverfahren für organische Stoffe nach Anspruch 5, **dadurch gekennzeichnet, dass** in Schritt (A) Materialien kontinuierlich in den ersten Reaktor (2) eindringen; und in Schritt (C) Materialien kontinuierlich aus dem ersten Reaktor (2) abgelassen werden.

8. Fermentationsverfahren für organische Stoffe nach Anspruch 5, **dadurch gekennzeichnet, dass** in Schritt (A) Materialien durch die Fördervorrichtung (1) in einem Intervall von 1-60 min zugeführt werden, wobei in Schritt (C) der Materialstrom mit hohem Feststoffanteil aus dem ersten Reaktor (2) durch die Auslassvorrichtung (4) für hohe Feststoffanteile in einem Intervall von 1-60 min abgelassen wird.

9. Fermentationsverfahren für organische Stoffe nach Anspruch 5, **dadurch gekennzeichnet, dass** der Feststoffanteil des ersten Reaktors (2) auf einem Wert von über 12 % und der Feststoffanteil des zweiten Reaktors (7) auf einem Wert von unter 12 % gehalten wird.

10. Fermentationsverfahren für organische Stoffe nach Anspruch 5, **dadurch gekennzeichnet, dass** die Temperatur des ersten Reaktors (2) und des zweiten Reaktors (7) zwischen 30-55°C gehalten wird.

11. Fermentationsverfahren für organische Stoffe nach Anspruch 5, **dadurch gekennzeichnet, dass** Schritt (D) ferner die folgenden Teilschritte umfasst:
(E) die Materialien in dem zweiten Reaktor (7) werden durch die erste Auslassvorrichtung (9) abgelassen und dazu getrieben, zum dritten Reaktor zu fließen;
(F) die Materialien in dem dritten Reaktor werden durch den zweiten Rücklauf in den erste Reaktor (2) zurückgeführt, um mit den ursprünglichen Materialien in dem ersten Reaktor (2) gemischt zu werden, und anschließend wird Schritt (B) wiederholt.

## Revendications

1. Équipement de fermentation utilisé pour les matières organiques, **caractérisé en ce qu'**il comprend un dispositif d'alimentation (1), un premier dispositif de réaction (2), un dispositif de pression différentielle (3), un dispositif d'évacuation de haute teneur en solides (4), un dispositif d'évacuation de basse teneur en solides (5), une première pompe à cycle (6), un deuxième dispositif de réaction (7), un premier dispositif de recirculation (8), un premier dispositif d'évacuation (9), un dispositif de collecte (10), et un dispositif d'agitation (12), un troisième dispositif de recirculation (18), dans lequel le dispositif d'alimentation (1) est raccordé au premier dispositif de réaction (2) ; le dispositif d'agitation (12) est agencé dans le premier dispositif de réaction (2) ; le dispositif de pression différentielle (3) est installé dans le premier dispositif de réaction (2) ; l'évacuation de haute teneur en solides et le dispositif d'évacuation de basse teneur en solides sont respectivement raccordés au premier dispositif de réaction (2) ; la première pompe à cycle (6) est agencée entre le dispositif d'évacuation de basse teneur en solides et le deuxième dispositif de réaction (7) ; le premier dispositif de recirculation (8) est agencé entre le premier dispositif de réaction (2) et le deuxième dispositif de réaction (7) ; le premier dispositif d'évacuation (9) est raccordé au deuxième dispositif de réaction (7) ; et le dispositif de collecte (10) est raccordé respectivement au premier dispositif de réaction (2) et au deuxième dispositif de réaction (7) pour collecter le biogaz généré dans lesdits premier et deuxième dispositifs de réaction (2, 7) ; le troisième dispositif de recirculation (18) est raccordé au premier dispositif de réaction (2) et au dispositif d'évacuation de haute teneur en solides (4), pour remettre en circulation les matières à haute teneur en solides dans le premier dispositif de réaction (2) pour amener les matières à réagir complètement, le dispositif de pression différentielle (3) est une structure à vis d'Archimède, ou un dispositif à piston hydraulique, ou une structure à compresseur.

2. Équipement de fermentation utilisé pour les matières organiques selon la revendication 1, **caractérisé en ce qu'**il comprend également un dispositif de chauffage.

3. Équipement de fermentation utilisé pour les matières organiques selon la revendication 1, **caractérisé en ce qu'**il comprend également un troisième dispositif de réaction, un deuxième dispositif de recirculation, et un deuxième dispositif d'évacuation, dans lequel le troisième dispositif de réaction est raccordé au deuxième dispositif de réaction (7) par l'intermédiaire du premier dispositif d'évacuation (9) ; le deuxième dispositif de recirculation est agencé entre le troisième dispositif de réaction et le premier dispositif de réaction (2) ; et le deuxième dispositif d'évacuation est raccordé au troisième dispositif de réaction.

4. Équipement de fermentation utilisé pour un dispositif de matières organiques selon la revendication 1, **caractérisé en ce qu'**il comprend également un dispositif de dosage, dans lequel le dispositif de dosage est agencé à n'importe quelle position dans le premier dispositif de réaction (2), le deuxième dispositif de réaction (7), le troisième dispositif de réaction, le premier dispositif de recirculation (8) ou le deuxième dispositif de recirculation.

5. Procédé de fermentation utilisé pour les matières organiques, comprenant les étapes suivantes :
(A) le transport de matières dans le premier dispositif de réaction (2) par le dispositif d'alimentation (1) pour effectuer une réaction de fermentation ;
(B) l'introduction d'un flux relatif de la phase solide et de la phase liquide dans le premier dispositif de réaction (2) avec le dispositif de pression différentielle (3) en créant des cycles d'excès de pression, de décompression et/ou de traction sur la phase solide par rapport à la phase liquide, le dispositif de pression différentielle (3) est une structure à vis d'Archimède ou un dispositif à piston hydraulique ;
(C) l'évacuation du flux de matières à haute teneur en solides à partir du premier dispositif de réaction (2) par le dispositif d'évacuation de haute teneur en solides (4) et ensuite le flux de matières à haute teneur en solides est amené à ré-entrer dans le premier dispositif de réaction (2) par le troisième dispositif de recirculation (18) pour être mélangé avec les matières d'origine dans le premier dispositif de réaction (2), l'évacuation du flux de matières à basse teneur en solides à partir du premier dispositif de réaction (2) par le dispositif d'évacuation de basse teneur en solides (5) et le pompage du flux de matières à basse teneur en solides dans le deuxième dispositif de réaction (7) par la première pompe à cycle (6) pour effectuer une réaction de digestion ;
(D) le retour par recirculation des matières dans le deuxième dispositif de réaction (7) vers le premier dispositif de réaction (2) par le premier dispositif de recirculation (8), le mélange des matières avec la matière d'origine dans le premier dispositif de réaction (2), et la répétition de l'étape (B) ; dans lequel il est en outre prévu de remettre en circulation les matières à haute teneur en solides dans le premier dispositif de réaction (2), par un troisième dispositif de recirculation (18), pour amener les matières à réagir.

6. Procédé de fermentation utilisé pour les matières organiques selon la revendication 5, **caractérisé en ce que** la durée de fonctionnement d'un cycle du dispositif de pression différentielle (3) est dans la plage de 1 à 60 minutes.

7. Procédé de fermentation utilisé pour les matières organiques selon la revendication 5, **caractérisé en ce que** dans l'étape (A), les matières entrent en continu dans le premier dispositif de réaction (2) ; et dans l'étape (C), les matières sont évacuées en continu à partir du premier dispositif de réaction (2).

8. Procédé de fermentation utilisé pour les matières organiques selon la revendication 5, **caractérisé en ce que** dans l'étape (A), les matières sont transportées par le dispositif d'alimentation (1) à un intervalle de 1 à 60 minutes ; dans l'étape (C), le flux de matières à haute teneur en solides est évacué du premier dispositif de réaction (2) par le dispositif d'évacuation de haute teneur en solides (4) à un intervalle de 1 à 60 minutes.

9. Procédé de fermentation utilisé pour les matières organiques selon la revendication 5, **caractérisé en ce que** la teneur en solides du premier dispositif de réaction (2) est maintenue pour être supérieure à 12 %, et la teneur en solides du deuxième dispositif de réaction (7) est maintenue pour être inférieure à 12 %.

10. Procédé de fermentation utilisé pour les matières organiques selon la revendication 5, **caractérisé en ce que** la température du premier dispositif de réaction (2) et du deuxième dispositif de réaction (7) est maintenue entre 30 et 55 °C.

11. Procédé de fermentation utilisé pour les matières organiques selon la revendication 5, **caractérisé en ce que** l'étape (D) comprend également les sous-étapes suivantes :
(E) les matières dans le deuxième dispositif de réaction (7) sont évacuées et amenés à s'écouler vers le troisième dispositif de réaction par le premier dispositif d'évacuation (9) ;
(F) les matières dans le troisième dispositif de réaction sont amenées à refluer dans le premier dispositif de réaction (2) par le deuxième dispositif de recirculation pour être mélangées avec les matières d'origine dans le premier dispositif de réaction (2), et ensuite l'étape (B) est répétée.
